(19) **Europäisches Patentamt / European Patent Office / Office européen des brevets**

(11) **EP 4 717 274 A1**

(12) **EUROPEAN PATENT APPLICATION**
published in accordance with Art. 153(4) EPC

(43) Date of publication:
01.04.2026 Bulletin 2026/14

(21) Application number: 23938438.1

(22) Date of filing: 23.05.2023

(51) International Patent Classification (IPC):
A61K 45/00 (2006.01)     A61K 31/407 (2006.01)
A61K 31/44 (2006.01)     A61K 31/4402 (2006.01)
A61K 31/47 (2006.01)     A61K 31/506 (2006.01)
A61K 31/517 (2006.01)     A61K 35/742 (2015.01)
A61K 39/395 (2006.01)     A61P 35/00 (2006.01)
A61P 43/00 (2006.01)     A61P 37/02 (2006.01)

(52) Cooperative Patent Classification (CPC):
A61K 31/407; A61K 31/44; A61K 31/4402;
A61K 31/47; A61K 31/506; A61K 31/517;
A61K 35/742; A61K 39/395; A61K 45/00;
A61P 35/00; A61P 37/02; A61P 43/00

(86) International application number:
PCT/JP2023/019078

(87) International publication number:
WO 2024/241468 (28.11.2024 Gazette 2024/48)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicants:
• Miyarisan Pharmaceutical Co., Ltd.
Sakaki-machi
Hanishina-gun
Nagano 389-0682 (JP)
• Osaka Prefectural Hospital Organization
Chuo-ku
Osaka-shi, Osaka 541-8567 (JP)

(72) Inventors:
• MIYOSHI, Norikatsu
Osaka-shi, Osaka 541-8567 (JP)
• ITO, Aya
Osaka-shi, Osaka 541-8567 (JP)
• TOJO, Ayaka
Osaka-shi, Osaka 541-8567 (JP)
• TAKAHASHI, Motomichi
Tokyo 114-0016 (JP)
• OKA, Kentaro
Tokyo 114-0016 (JP)
• HAYASHI, Atsushi
Tokyo 114-0016 (JP)
• KUDO, Hayami
Tokyo 114-0016 (JP)

(74) Representative: J A Kemp LLP
80 Turnmill Street
London EC1M 5QU (GB)

(54) **COMBINATION MEDICINE FOR TREATING CANCER**

(57) [Problem] Provided is a novel pharmaceutical combination for cancer treatment.

[Solution] A pharmaceutical combination for cancer treatment, including a tyrosine kinase inhibitor and Clostridium butyricum MIYAIRI 588 (FERM BP-2789) or a culture thereof.

Fig. 1

**Description**

**TECHNICAL FIELD**

The present invention relates to a pharmaceutical combination for cancer treatment.

**BACKGROUND ART**

Probiotics are defined as "live microorganisms that beneficially influence the health of hosts by improving the balance of their intestinal flora", and their practical clinical application has also been actively attempted in the medical field.
In addition, probiotics are expected to enhance the therapeutic effects on cancer patients when used in combination with chemotherapy, such as anticancer agents.
For example, Patent Literature 1 describes that the therapeutic effects of an immune checkpoint inhibitor can be enhanced by administering to a cancer patient the immune checkpoint inhibitor and Clostridium butyricum MIYAIRI 588 (FERM BP-2789).

Citation List

Patent Literature

Patent Literature 1: WO 2021/216618

**SUMMARY OF INVENTION**

An object of the present invention is to provide a novel pharmaceutical combination for cancer treatment.
The present inventors have surprisingly found that antitumor effects can be improved by combining Clostridium butyricum MIYAIRI 588 (FERM BP-2789) with a tyrosine kinase inhibitor. Accordingly, the present inventors have completed the present invention based on this finding.
Thus, according to an aspect of the present invention, provided is a pharmaceutical combination for cancer treatment, containing a tyrosine kinase inhibitor and Clostridium butyricum MIYAIRI 588 (FERM BP-2789) or a culture thereof.

**BRIEF DESCRIPTION OF DRAWINGS**

Fig. 1 is graphs showing the changes in tumor volume over time in the respective groups in Test Example 1.

Fig. 2 is graphs showing the tumor volumes at the observation time points (Day 4, Day 8, Day 11, Day 15, and Day 18) in the respective groups in Test Example 1.

Fig. 3 is graphs showing the Kaplan-Meier curves in which a tumor volume of 1000 mm$^3$ is defined as an event in Test Example 1.

**DESCRIPTION OF EMBODIMENTS**

Embodiments according to an aspect of the present invention will now be described. The present invention is not limited to only the following embodiments.
As used herein, the expression "X to Y" that indicates a range means "X or more and Y or less". In addition, unless otherwise specified, operations, measurements of physical properties, and the like are performed under the conditions of room temperature (20 to 25°C) and relative humidity of 40 to 50% RH.
As used herein, "Clostridium butyricum MIYAIRI 588 (FERM BP-2789)" is also referred to simply as "CBM588".
A first aspect of the present invention is a pharmaceutical combination for cancer treatment, including a tyrosine kinase inhibitor and CBM588 or a culture thereof.
A second aspect of the present invention is a method for treating cancer, including administering to a subject an effective amount of a tyrosine kinase inhibitor and an effective amount of CBM588 or a culture thereof in combination.
A third aspect of the present invention is a pharmaceutical composition for use in cancer treatment, including a combination of a tyrosine kinase inhibitor and CBM588 or a culture thereof.

A fourth aspect of the present invention is a pharmaceutical composition for cancer treatment, including CBM588 or a culture thereof administered in combination with a tyrosine kinase inhibitor.

A fifth aspect of the present invention is a pharmaceutical composition for cancer treatment, including a tyrosine kinase inhibitor administered in combination with CBM588 or a culture thereof.

A sixth aspect of the present invention is a kit for cancer treatment, including a tyrosine kinase inhibitor and CBM588 or a culture thereof.

A seventh aspect of the present invention is CBM588 or a culture thereof for use together with a tyrosine kinase inhibitor in a method for treating cancer.

An eighth aspect of the present invention is a tyrosine kinase inhibitor for use together with CBM588 or a culture thereof in a method for treating cancer.

A ninth aspect of the present invention is use of a tyrosine kinase inhibitor and/or CBM588 or a culture thereof in a medicament for use in a method for treating cancer comprising administering to a subject a tyrosine kinase inhibitor and CBM588 or a culture thereof.

The present invention can improve the antitumor effect, in particular, the reducing effect on tumor volume by administering a tyrosine kinase inhibitor and CBM588 or a culture thereof in combination, compared to administering a tyrosine kinase inhibitor alone, thereby allowing the significant increase in the survival rate of the subject.

The tyrosine kinase inhibitor according to the present invention is used in combination with Clostridium butyricum MIYAIRI 588 (FERM BP-2789) or a culture thereof for cancer treatment.

The tyrosine kinase inhibitor refers to an agent exhibiting the antitumor effect by inhibiting cellular tyrosine kinases, enzymes involved in many cell functions such as cell signaling, growth, and division.

The tyrosine kinase inhibitor according to the present invention is not particularly limited, and any conventionally known tyrosine kinase inhibitors can be used. The tyrosine kinase inhibitor is preferably at least one selected from the group consisting of an EGFR tyrosine kinase inhibitor, a c-kit receptor tyrosine kinase inhibitor, a BCR-ABL tyrosine kinase inhibitor, and a multikinase inhibitor, and is more preferably a multikinase inhibitor.

Specific examples of the tyrosine kinase inhibitor according to the present invention include, but not particularly limited to, at least one selected from the group consisting of imatinib, dasatinib, nilotinib, afatinib, erlotinib, osimertinib, gefitinib, axitinib, cabozantinib, sunitinib, sorafenib, pazopanib, regorafenib, and lenvatinib, and more preferably at least one selected from the group consisting of axitinib, cabozantinib, sunitinib, sorafenib, pazopanib, regorafenib, and lenvatinib.

The Clostridium butyricum MIYAIRI 588 (FERM BP-2789) or a culture thereof according to the present invention is used in combination with a tyrosine kinase inhibitor for cancer treatment.

CBM588 is a spore-forming bacterium, and when in a spore state, it is resistant to various external environments. Therefore, when CBM588 is orally administered to a human or an animal in the form of spores, CBM588 does not die completely even when it comes into contact with digestive fluid such as gastric acid, intestinal fluid, and bile acid, and it can then reach the fermenting sites from the lower part of small intestine to the large intestine to grow.

In addition, CBM588 has been widely commercially available as live bacterial agents, feed additives, and food, and it has shown no side effects even when administered to a mammal such as a human or a domestic animal for an extended period of time, ensuring a high safety level.

CBM588 was deposited on May 1, 1981 as FERM BP-2789 at Fermentation Research Institute, Agency of Industrial Science and Technology, Ministry of International Trade and Industry (currently International Patent Organism Depositary, National Institute of Technology and Evaluation) (Unit 120, 2-5-8 Kazusakamatari, Kisarazu City, Chiba Prefecture, 292-0818, Japan), and transferred to an international depository authority under the Budapest Treaty on March 6, 1990, where it is deposited under the accession number: FERM BP-2789.

CBM588 is commercially available from Miyarisan Pharmaceutical Co., Ltd. as a probiotic agent, which shows no side effects even when administered to a human or an animal for an extended period of time. In a preferred embodiment, CBM588 is a viable bacterium.

As used herein, a culture of CBM588 means a culture solution in which Clostridium butyricum was cultured, a residue containing a bacterium obtained by centrifuging the culture solution, and a dried product of the residue.

The culture of CBM588 is obtained by a known method for culturing a microorganism, for example, the method disclosed in JP 08-252088 A. An embodiment thereof is shown below: CBM588 is inoculated into a medium containing 1.0% (w/v) peptone, 1.0% (w/v) yeast extract, 1.0% (w/v) corn starch, and 0.2% (w/v) precipitated calcium carbonate so as to be $10^5$ to $10^6$ cells/mL, and subjected to static culture for 48 hours at 37°C to obtain a "culture solution of CBM588". Next, the culture solution thus obtained is centrifuged (2,000 to 6,000 g × 10 to 30 minutes) to separate the "residue containing a bacterium obtained by centrifuging the culture solution", and this residue is dried by air drying or the like at 0 to 80°C, preferably 10 to 20°C for 1 to 24 hours, preferably 5 to 18 hours, or dried under reduced pressure at 0 to 80°C, preferably 10 to 20°C at 0.05 to 500 Torr (7 Pa to 66.7 kPa), preferably 1 to 100 Torr (133 Pa to 13.3 kPa) for 1 to 24 hours, preferably 2 to 15 hours to obtain a "dried product of the residue". To obtain a dried product, spray drying, freeze drying, or the like can be used.

A medium to be used for culturing CBM588 according to the present invention may be a synthetic medium or a natural medium, as long as it contains a carbon source that CBM588 can assimilate, an appropriate amount of nitrogen source, an inorganic salt, and other nutrients such as vitamins.

For example, the carbon source used in the medium according to the present invention is not particularly limited as long as CBM588 can assimilate the carbon source. The carbon source is not necessarily limited to sugar; however, in consideration of the growth of bacterial cells, a sugar or sugar-containing source that CBM588 can use is preferably used. Specific examples of usable carbon sources include cellobiose, glucose, fructose, galactose, lactose, maltose, mannose, melibiose, raffinose, salicin, starch, sucrose, trehalose, xylose, dextrin, and molasses, in consideration of assimilation property. Among these carbon sources, starch, glucose, fructose, sucrose, and molasses are preferably used. One or two or more of the above carbon sources can be selected for use in consideration of CBM588. In such a case, the concentration of the carbon source added varies depending on the type of carbon source used, the medium composition other than the carbon source of the medium used, and the like, and it is usually 0.5 to 5% (w/v), and preferably 2 to 4% (w/v).

In addition, examples of the nitrogen source and vitamins include meat extract, peptone, yeast extract, soybean and wheat hydrolysate such as seasoning liquid, soybean powder, milk casein, casamino acid, various amino acids, corn steep liquor, other organic nitride compounds such as hydrolysates of animals, plants and microorganisms, and ammonium salts such as ammonium sulfate. Among these nitrogen sources, peptone, yeast extract, meat extract, corn steep liquor, and seasoning liquid are preferably used. One or two or more of the above nitrogen sources and vitamins can be selected for use to improve the growth of CBM588. In such a case, the concentration of the nitrogen source added varies depending on the type of strain used, the type of nitrogen source, the medium composition other than the nitrogen source of the medium used, and the like; and when using peptone that contains a large amount of nitrogen sources, it is usually 0.5 to 4% (w/v), and preferably 1 to 3% (w/v); when using seasoning liquid or corn steep liquor that contains a large amount of nitrogen sources and vitamins, it is usually 0.5 to 5% (w/v), and preferably 1 to 4% (w/v); and when using yeast extract or meat extract that contains a large amount of vitamins, it is usually 0.5 to 4% (w/v), and preferably 1 to 3% (w/v).

Further, as the inorganic salts, one or two or more can be used that are selected from phosphates, hydrochlorides, sulfates, butyrates, propionates, acetates, and the like, for example, magnesium, manganese, calcium, sodium, potassium, molybdenum, strontium, boron, copper, iron, tin, and zinc. In addition, antifoaming agents, vegetable oils, surfactants, blood and blood components, agents such as antibiotics, bioactive substances such as plant or animal hormones, and the like can be appropriately added into the medium, as necessary.

Since CBM588 is obligate anaerobic, as the culturing conditions in the present invention, CBM588 is required to be cultured in anaerobic conditions by not airing, by airing nitrogen or carbon dioxide, by adding a reducing agent to the medium to lower redox potentials, or the like. The culturing conditions in such a case are not particularly limited, as long as the conditions are appropriately selected according to the medium composition and the culturing method used, and they allow CBM588 to grow. Specifically, the culturing temperature is usually 20 to 42°C, and preferably 35 to 40°C.

In addition, in the present invention, since the growth of CBM588 is facilitated by neutralizing the acids produced during the culture with alkalis, the culture of CBM588 preferably contains adding calcium carbonate to the medium in advance. In such a case, the amount of calcium carbonate added is usually 0.1 to 4% (w/v), and preferably 0.2 to 2.5% (w/v). Alternatively, it is also preferable to perform the above neutralizing step by controlling the pH of the medium within a set pH range using an aqueous alkali solution such as sodium hydroxide, sodium bicarbonate, sodium carbonate, potassium hydroxide, and potassium carbonate. Note that when an aqueous alkali solution is used, the "set pH" means a pH of the medium which has been set in advance during the culture period, and the "set pH range" means an acceptable pH range during the culture period, expressed generally as the set pH $\pm$ tolerance. According to the present invention, the set pH is usually set within a range of 5.0 to 7.5, preferably a range of 5.5 to 6.5, and the set pH range is the set pH $\pm$ 0.5, desirably the set pH $\pm$ 0.2.

Note that, in the present invention, the pH of the medium during the culture is close to neutral, and more preferably 6.5 to 7.5 when CBM588 is inoculated. Note that, when an aqueous alkali solution is used, it is preferable to keep the pH within the set pH range while gently stirring the solution to prevent oxygen from being mixed therein. By controlling the pH at the time of inoculation and during the growth of CBM588 in this way, the bacterial density can be dramatically increased.

In the culture according to the present invention, the initial culture concentration of CBM588 is not particularly limited as long as it is within the range where CBM588 can grow, and specifically, it is usually $10^4$ to $10^7$ cells/mL, and preferably $10^5$ to $10^6$ cells/mL.

The culture of CBM588 thus obtained can improve the antitumor effect when used in combination with a tyrosine kinase inhibitor.

In the present invention, an immune checkpoint inhibitor can be used in combination with a tyrosine kinase inhibitor and CBM588 or a culture thereof for cancer treatment.

The immune checkpoint inhibitor inhibits immune checkpoint functions of a receptor or a ligand, and examples thereof include antagonists of inhibitory receptors and agonists of co-stimulatory immune checkpoint receptors.

The term "antagonist" encompasses various substances that interfere with receptor activation through binding of a receptor and a ligand. Examples thereof can include substances that interfere with the bond between the receptor and the ligand by binding to the receptor, and substances that interfere with the bond between the receptor and the ligand by binding to the ligand.

Examples of the antagonists of inhibitory immune checkpoints include antagonistic antibodies that bind to inhibitory immune checkpoint molecules (inhibitory receptors or ligands for the receptors), soluble polypeptides that are designed based on inhibitory immune checkpoint ligands and that do not activate receptors, or vectors capable of expressing the polypeptides.

The immune checkpoint inhibitor according to the present invention is not particularly limited, and any conventional known immune checkpoint inhibitors can be used. The immune checkpoint inhibitor is preferably at least one selected from the group consisting of a PD-1 inhibitor, a PD-L1 inhibitor, and CTLA-4 inhibitor, and is more preferably a PD-1 inhibitor.

Specific examples of the immune checkpoint inhibitor according to the present invention include, but not particularly limited to, at least one selected from the group consisting of nivolumab, ipilimumab, pembrolizumab, cemipilimab, durvalumab, daclizumab, avelumab, and atezolizumab, and more preferably at least one selected from the group consisting of nivolumab, pembrolizumab, and cemipilimab.

Note that the present invention includes the following embodiments:

a pharmaceutical combination for cancer treatment, including a tyrosine kinase inhibitor, CBM588, and an immune checkpoint inhibitor;

a method for treating cancer, including administering to a subject an effective amount of tyrosine kinase inhibitor, an effective amount of CBM588 or a culture thereof, and an effective amount of immune checkpoint inhibitor in combination;

a pharmaceutical composition for use in cancer treatment, including a combination of a tyrosine kinase inhibitor, CBM588 or a culture thereof, and an immune checkpoint inhibitor;

a pharmaceutical composition for cancer treatment, including CBM588 or a culture thereof administered in combination with a tyrosine kinase inhibitor and an immune checkpoint inhibitor;

a pharmaceutical composition for cancer treatment, including a tyrosine kinase inhibitor administered in combination with CBM588 or a culture thereof and an immune checkpoint inhibitor;

a kit for cancer treatment, including a tyrosine kinase inhibitor, CBM588 or a culture thereof, and an immune checkpoint inhibitor;

CBM588 or a culture thereof for use together with a tyrosine kinase inhibitor and an immune checkpoint inhibitor in a method for treating cancer; and

a tyrosine kinase inhibitor for use together with CBM588 or a culture thereof, and an immune checkpoint inhibitor in a method for treating cancer.

In the present invention, the type of "cancer" to be treated is not particularly limited. Examples of the cancer include oral cavity cancer, pharyngeal cancer, esophageal cancer, gastric cancer, hepatic cancer, gallbladder cancer, biliary tract cancer, spleen cancer, colorectal cancer, small intestinal cancer, duodenal cancer, colon cancer, colon adenocarcinoma, rectal cancer, pancreatic cancer, liver cancer, bladder cancer, renal cell cancer, non-small-cell lung cancer, small cell lung cancer, malignant melanoma (melanoma), sarcoma, lymphoma, breast cancer, cervical cancer, endometrial cancer, ovarian cancer, testicular cancer, prostate cancer, head and neck cancer, and metastatic cancers thereof. The cancer is preferably at least one selected from the group consisting of colorectal cancer, metastatic renal cell cancer (mRCC), non-small-cell lung cancer, melanoma, sarcoma, lymphoma, breast cancer, bladder cancer, cervical cancer, head and neck cancer, liver cancer, gastric cancer, and rectal cancer.

As used herein, the term "effective amount" means an amount of active ingredient (i.e., tyrosine kinase inhibitor, CBM588, or immune checkpoint inhibitor) that is at least required to exert desired effects such as the treatment of cancer, in particular, the improvement of antitumor effect.

As used herein, the term "subject" is, but not particularly limited to, a mammal or a bird, and preferably a mammal or a bird affected by cancer or potentially affected by cancer. Here, the mammals encompass both primates such as humans, monkeys, gorillas, chimpanzees, and orangutans, and non-human mammals such as mice, rats, hamsters, guinea pigs, rabbits, dogs, cats, pigs, cows, horses, sheep, camels, and goats. Examples of the birds include chickens, quails, and pigeons. Among these, humans are preferred.

In the present invention, a tyrosine kinase inhibitor, CBM588 or a culture thereof, and, as necessary, an immune checkpoint inhibitor are administered to a subject in combination (combined administration).

Administering in combination (combined administration) includes simultaneously administering respective components, and sequentially administering respective components at predetermined intervals over the treatment period (combination therapy). The routes and means of administering respective components that are administered in combination may be the same or different.

The dosages and administration method of the tyrosine kinase inhibitor, CBM588 or a culture thereof, and the immune checkpoint inhibitor can be appropriately determined in consideration of the type of cancer, the symptoms, age, sex, body weight, and conditions of the subject, and the like.

The dosage of the tyrosine kinase inhibitor is, for example, about 1 to about 2400 mg/kg body weight/day in terms of the active ingredient. The tyrosine kinase inhibitor can be administered daily, every other day, or every few days, divided into 1 to 6 doses per day. The method for administering a tyrosine kinase inhibitor is not particularly limited, and examples thereof include oral administration, and parenteral administration including intravenous injection, intraarterial injection, subcutaneous injection, intradermal injection, intraperitoneal injection, intramuscular injection, intrathecal injection, transdermal administration, or transdermal absorption, and is preferably oral administration. The dosage of the tyrosine kinase inhibitor is 3 mg/kg body weight/day (5 times a week), and the administration method is oral administration in Examples described later.

The dosage of CBM588 or a culture thereof is, for example, 0.1 to 1000 mg/kg body weight/day in terms of the active ingredient. CBM588 or a culture thereof can be administered daily, every other day, or every few days, divided into 1 to 6 doses per day. The method for administering CBM588 or a culture thereof is not particularly limited, and examples thereof include oral administration, and parenteral administration including intravenous injection, intraarterial injection, subcutaneous injection, intradermal injection, intraperitoneal injection, intramuscular injection, intrathecal injection, transdermal administration, or transdermal absorption, and is preferably oral administration. The dosage of CBM588 is about 43 mg/kg body weight/day ($1 \times 10^8$ cfu (about 1.3 mg)/100 $\mu$L/animal), and the administration method is oral administration in Examples described later.

The dosage of the immune checkpoint inhibitor is, for example, 0.1 to 20 mg/kg body weight/day in terms of the active ingredient, and preferably 2 to 3 mg/kg body weight/day. The immune checkpoint inhibitor can be administered daily, every other day, or every few days, divided into 1 to 6 doses per day. The method for administering an immune checkpoint inhibitor is not particularly limited, and examples thereof include oral administration, and parenteral administration including intravenous injection, intraarterial injection, subcutaneous injection, intradermal injection, intraperitoneal injection, intramuscular injection, intrathecal injection, transdermal administration, or transdermal absorption, and is preferably intraperitoneal administration or intravenous injection. The dosage of the immune checkpoint inhibitor is 10 mg/kg body weight/day (5 times every 3 days), and the administration method is intraperitoneal administration in Examples described later.

In the present invention, the tyrosine kinase inhibitor, CBM588 or a culture thereof, and the immune checkpoint inhibitor may be in the form of a pharmaceutical combination, or each may be in the form of a separate medicament (or pharmaceutical composition).

The pharmaceutical combination may be in the form of a combination preparation containing respective components in the same composition, or in the form of a single package (i.e., the form of a kit) suitable for combination administration after each component is separately prepared.

In the present invention, in addition to the above components, additives acceptable for formulation can be further used. Examples of the additive can include excipients, stabilizers, preservatives, humectants, emulsifiers, lubricants, sweeteners, colorants, flavorings, buffers, antioxidants, pH adjusters, binders, thickeners, dispersants, suspending agents, disintegrants, bacteriostatic agents, and surfactants.

In the present invention, the dosage forms of the pharmaceutical combination, the pharmaceutical composition, the tyrosine kinase inhibitor, CBM588 or a culture thereof, and the immune checkpoint inhibitor are not particularly limited and may be appropriately determined; and examples thereof include tablets, powders, fine granules, granules, capsules, pills, sustained release agents, solutions, suspensions, emulsions, lotions, injections, infusions, external preparations, suppositories, and patches.

The following are exemplary embodiments of the present invention.

[1] A pharmaceutical combination for cancer treatment, including a tyrosine kinase inhibitor and Clostridium butyricum MIYAIRI 588 (FERM BP-2789) or a culture thereof.

[2] The pharmaceutical combination for cancer treatment according to [1], wherein the Clostridium butyricum MIYAIRI 588 is a viable bacterium.

[3] The pharmaceutical combination for cancer treatment according to [1] or [2], wherein the tyrosine kinase inhibitor is at least one selected from the group consisting of an EGFR tyrosine kinase inhibitor, a c-kit receptor tyrosine kinase inhibitor, a BCR-ABL tyrosine kinase inhibitor, and a multikinase inhibitor.

[4] The pharmaceutical combination for cancer treatment according to any of [1] to [3], wherein the tyrosine kinase inhibitor is at least one selected from the group consisting of imatinib, dasatinib, nilotinib, afatinib, erlotinib, osimertinib, gefitinib, axitinib, cabozantinib, sunitinib, sorafenib, pazopanib, regorafenib, and lenvatinib.

[5] The pharmaceutical combination for cancer treatment according to any of [1] to [4], further including an immune checkpoint inhibitor.

[6] The pharmaceutical combination for cancer treatment according to [5], wherein the immune checkpoint inhibitor is at least one selected from the group consisting of a PD-1 inhibitor, a PD-L1 inhibitor, and CTLA-4 inhibitor.

[7] The pharmaceutical combination for cancer treatment according to [5] or [6], wherein the immune checkpoint inhibitor is at least one selected from the group consisting of nivolumab, ipilimumab, pembrolizumab, cemipilimab, durvalumab, daclizumab, avelumab, and atezolizumab.

[8] The pharmaceutical combination for cancer treatment according to any of [1] to [7], wherein the cancer is at least one selected from the group consisting of colorectal cancer, metastatic renal cell cancer (mRCC), non-small-cell lung cancer, melanoma, sarcoma, lymphoma, breast cancer, bladder cancer, cervical cancer, head and neck cancer, liver cancer, gastric cancer, and rectal cancer.

[9] A method for treating cancer, including administering to a subject an effective amount of tyrosine kinase inhibitor and an effective amount of Clostridium butyricum MIYAIRI 588 (FERM BP-2789) or a culture thereof in combination.

[10] A pharmaceutical composition for use in cancer treatment, including a combination of a tyrosine kinase inhibitor and Clostridium butyricum MIYAIRI 588 (FERM BP-2789) or a culture thereof.

[11] A pharmaceutical composition for cancer treatment, including Clostridium butyricum MIYAIRI 588 (FERM BP-2789) or a culture thereof administered in combination with a tyrosine kinase inhibitor.

[12] A pharmaceutical composition for cancer treatment, including a tyrosine kinase inhibitor administered in combination with Clostridium butyricum MIYAIRI 588 (FERM BP-2789) or a culture thereof.

[13] A kit for cancer treatment, including a tyrosine kinase inhibitor and Clostridium butyricum MIYAIRI 588 (FERM BP-2789) or a culture thereof.

[14] Clostridium butyricum MIYAIRI 588 (FERM BP-2789) or a culture thereof for use together with a tyrosine kinase inhibitor in a method for treating cancer.

[15] A tyrosine kinase inhibitor for use together with Clostridium butyricum MIYAIRI 588 (FERM BP-2789) or a culture thereof in a method for treating cancer.

[16] Use of a tyrosine kinase inhibitor and/or Clostridium butyricum MIYAIRI 588 (FERM BP-2789) or a culture thereof in a medicament for use in a method for treating cancer comprising administering to a subject a tyrosine kinase inhibitor and Clostridium butyricum MIYAIRI 588 (FERM BP-2789) or a culture thereof.

Examples

The present invention will be described using specific examples below; however, the present invention is not limited to these. Unless otherwise specified, the operation was performed at room temperature (25°C).
Animal experiments using mice were conducted by obtaining approval from the Animal Experiment Committee regarding animal experimentation of the Osaka International Cancer Institute (approval number: 23022121).

<Test Example 1>

BALB/c mice (7-week-old, male/female, purchased from CLEA Japan, Inc., SPF) were subjected to quarantine and habituation for a period of 7 days, and all the individuals were confirmed to be in good health, and no weight loss followed by being subjected to a test. Mice were allowed to freely consume feed and water in a rearing environment of 12-hour lighting, a temperature of 20 to 26°C, and humidity of 30 to 70%.

$1 \times 10^6$ cells of a mouse colorectal cancer cell line (CT26) were collected and mixed with 80 μL of highly concentrated Matrigel, and the mixture was subcutaneously injected into mice. From Day 21 after subcutaneous injection or the time of confirmation of tumor formation by palpation, whichever came first, the administration of tyrosine kinase inhibitor (TKI), immune checkpoint inhibitor (ICI), and Clostridium butyricum MIYAIRI 588 (FERM BP-2789) was conducted in 5 groups as follows: TKI group, TKI + CBM group, ICI group, TKI + ICI group, and TKI + ICI + CBM group (n = 7 (TKI group, TKI +CBM group) and n = 6 (ICI group, TKI + ICI group, TKI + ICI + CBM group)). The administration of TKI, ICI, and CBM588 was not conducted in the control group (n = 7).

Mice that had been subjected to the test were euthanized on Day 28 from the start of the administration, or when the tumor short diameter reached 10 mm or when the tumor mass reached 10% of their body weight, which was considered a humane endpoint. Also, mice with tumor volumes exceeding 1500 mm$^3$ by Day 11 from the start of the administration were excluded.

Details of the agents used and their dosage and administration are shown below.

TKI: 30 μg/g of regorafenib (#R0142, Tokyo Chemical Industry Co., Ltd.), 100 μL once a day (maximum amount of 1000 μL), administered orally for 9 days

ICI: 5 μg/g of anti-CD279 antibody (J43; BE-00332, Iwai Chemicals Company, Ltd.), 100 μL every 3 days (maximum amount of 1600 μL), administered intraperitoneally (the administration continued until the end of the observation period)

CBM: $1 \times 10^8$ cfu/100 μL of raw materials of MiyaBM (Miyarisan Pharmaceutical Co., Ltd.), 100 μL per once, administered orally 3 times a week.

The tumor short diameters (mm) and tumor long diameters (mm) were measured on Day 4, Day 8, Day 11, Day 15, and Day 18 from the start of the administration to determine the tumor volumes according to the following equation:

$$\text{Tumor volume (mm}^3) = 1/2 \times \text{tumor short diameter} \times \text{tumor short diameter} \times \text{tumor long diameter}.$$

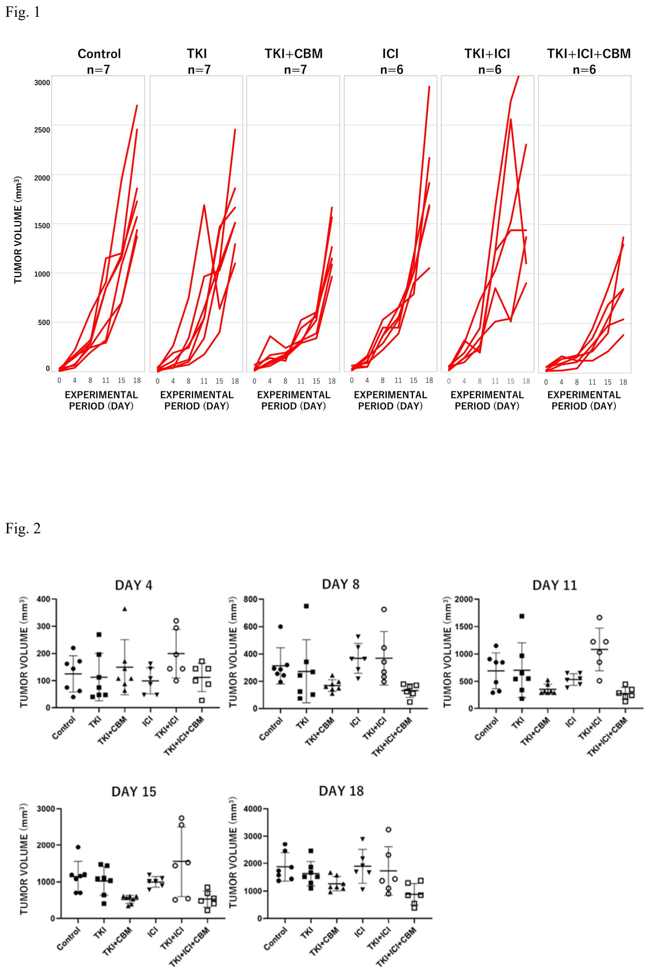

Results are shown in Figs. 1 and 2.

As shown in Figs. 1 and 2, it can be seen that combining the tyrosine kinase inhibitor with CBM588 can suppress the increase in tumor volume.

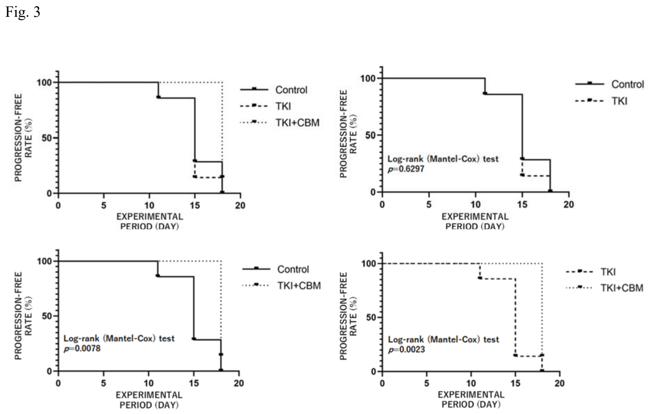

Further, Fig. 3 shows the Kaplan-Meier curves in which a tumor volume of 1000 mm$^3$ is defined as an event. As shown in Fig. 3, it can be seen that TKI + CBM group exhibits a significantly higher progression-free rate than the control group and TKI group by the log-rank test.

## Claims

1. A pharmaceutical combination for cancer treatment, comprising a tyrosine kinase inhibitor and Clostridium butyricum MIYAIRI 588 (FERM BP-2789) or a culture thereof.

2. The pharmaceutical combination for cancer treatment according to claim 1, wherein the Clostridium butyricum MIYAIRI 588 is a viable bacterium.

3. The pharmaceutical combination for cancer treatment according to claim 1, wherein the tyrosine kinase inhibitor is at least one selected from the group consisting of an EGFR tyrosine kinase inhibitor, a c-kit receptor tyrosine kinase inhibitor, a BCR-ABL tyrosine kinase inhibitor, and a multikinase inhibitor.

4. The pharmaceutical combination for cancer treatment according to claim 3, wherein the tyrosine kinase inhibitor is at least one selected from the group consisting of imatinib, dasatinib, nilotinib, afatinib, erlotinib, osimertinib, gefitinib, axitinib, cabozantinib, sunitinib, sorafenib, pazopanib, regorafenib, and lenvatinib.

5. The pharmaceutical combination for cancer treatment according to claim 1, further comprising an immune checkpoint inhibitor.

6. The pharmaceutical combination for cancer treatment according to claim 5, wherein the immune checkpoint inhibitor is at least one selected from the group consisting of a PD-1 inhibitor, a PD-L1 inhibitor, and CTLA-4 inhibitor.

7. The pharmaceutical combination for cancer treatment according to claim 6, wherein the immune checkpoint inhibitor is at least one selected from the group consisting of nivolumab, ipilimumab, pembrolizumab, cemipilimab, durvalumab, daclizumab, avelumab, and atezolizumab.

8. The pharmaceutical combination for cancer treatment according to claim 1, wherein the cancer is at least one selected from the group consisting of colorectal cancer, metastatic renal cell cancer (mRCC), non-small-cell lung cancer, melanoma, sarcoma, lymphoma, breast cancer, bladder cancer, cervical cancer, head and neck cancer, liver cancer, gastric cancer, and rectal cancer.

9. A method for treating cancer, comprising administering to a subject an effective amount of tyrosine kinase inhibitor and an effective amount of Clostridium butyricum MIYAIRI 588 (FERM BP-2789) or a culture thereof in combination.

10. A pharmaceutical composition for use in cancer treatment, comprising a combination of a tyrosine kinase inhibitor and Clostridium butyricum MIYAIRI 588 (FERM BP-2789) or a culture thereof.

11. A pharmaceutical composition for cancer treatment, comprising Clostridium butyricum MIYAIRI 588 (FERM BP-2789) or a culture thereof administered in combination with a tyrosine kinase inhibitor.

12. A pharmaceutical composition for cancer treatment, comprising a tyrosine kinase inhibitor administered in combination with Clostridium butyricum MIYAIRI 588 (FERM BP-2789) or a culture thereof.

13. A kit for cancer treatment, comprising a tyrosine kinase inhibitor and Clostridium butyricum MIYAIRI 588 (FERM BP-2789) or a culture thereof.

14. Clostridium butyricum MIYAIRI 588 (FERM BP-2789) or a culture thereof for use together with a tyrosine kinase inhibitor in a method for treating cancer.

15. A tyrosine kinase inhibitor for use together with Clostridium butyricum MIYAIRI 588 (FERM BP-2789) or a culture thereof in a method for treating cancer.

16. Use of a tyrosine kinase inhibitor and/or Clostridium butyricum MIYAIRI 588 (FERM BP-2789) or a culture thereof in a medicament for use in a method for treating cancer comprising administering to a subject a tyrosine kinase inhibitor and Clostridium butyricum MIYAIRI 588 (FERM BP-2789) or a culture thereof.

Fig. 1

Fig. 2

Fig. 3

## EP 4 717 274 A1

<table>
<tr><td colspan="2">INTERNATIONAL SEARCH REPORT</td><td colspan="2">International application No.<br>**PCT/JP2023/019078**</td></tr>
</table>

**A. CLASSIFICATION OF SUBJECT MATTER**

*A61K 45/00*(2006.01)i; *A61K 31/407*(2006.01)i; *A61K 31/44*(2006.01)i; *A61K 31/4402*(2006.01)i; *A61K 31/47*(2006.01)i; *A61K 31/506*(2006.01)i; *A61K 31/517*(2006.01)i; *A61K 35/742*(2015.01)i; *A61K 39/395*(2006.01)i; *A61P 35/00*(2006.01)i; *A61P 43/00*(2006.01)i; *A61P 37/02*(2006.01)n

FI: A61K45/00; A61K31/407; A61K31/44; A61K31/4402; A61K31/47; A61K31/506; A61K31/517; A61K35/742; A61K39/395 T; A61K39/395 U; A61P35/00; A61P43/00 121; A61P37/02

According to International Patent Classification (IPC) or to both national classification and IPC

**B. FIELDS SEARCHED**

Minimum documentation searched (classification system followed by classification symbols)

A61K45/00; A61K31/407; A61K31/44; A61K31/4402; A61K31/47; A61K31/506; A61K31/517; A61K35/742; A61K39/395; A61P35/00; A61P43/00; A61P37/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); PubMed; Science Direct

**C. DOCUMENTS CONSIDERED TO BE RELEVANT**

| Category* | Citation of document, with indication, where appropriate, of the relevant passages | Relevant to claim No. |
|---|---|---|
| X | JP 8-252088 A (NIPPO KAGAKU KK) 01 October 1996 (1996-10-01)<br>examples 1-5 | 14 |
| X | WO 2021/020554 A1 (MOMOTARO-GENE INC., NATIONAL UNIVERSITY CORPORATION OKAYAMA UNIVERSIRY) 04 February 2021 (2021-02-04)<br>claims | 15 |
| X | MEZA, L. A. et al. A phase I trial to evaluate the biologic effect of CBM588 (Clostridium butyricum) in combination with cabozantinib plus nivolumab for patients with metastatic renal cell carcinoma (mRCC). Journal of Clinical Oncology. 02 June 2022, vol. 40, issue 16, suppl., TPS4606, in particular, columns "background", "method"<br>columns "background", "method" | 1-16 |
| Y | WO 2021/216618 A1 (2021/216618) 28 October 2021 (2021-10-28)<br>claims | 1-16 |

☑ Further documents are listed in the continuation of Box C.    ☑ See patent family annex.

| | |
|---|---|
| *   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **02 August 2023** | **15 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| | International application No. |
|---|---|
| | **PCT/JP2023/019078** |

**C.    DOCUMENTS CONSIDERED TO BE RELEVANT**

| Category* | Citation of document, with indication, where appropriate, of the relevant passages | Relevant to claim No. |
|---|---|---|
| Y | DE ALMEIDA LEITE, Rodrigo Moises et al. Regorafenib - Five Years in Review. European Oncology & Haematology. 2020, vol. 16, no. 1, pp. 24-28<br>p. 24, column "abstract", etc. | 1-16 |
| A | JP 2009-269836 A (MIYARISAN PHARMACEUTICAL CO., LTD.) 19 November 2009 (2009-11-19)<br>claims, examples | 1-16 |

Form PCT/ISA/210 (second sheet) (January 2015)

## INTERNATIONAL SEARCH REPORT
**Information on patent family members**

International application No.

**PCT/JP2023/019078**

| Patent document cited in search report | | | Publication date (day/month/year) | Patent family member(s) | | | Publication date (day/month/year) |
|---|---|---|---|---|---|---|---|
| JP | 8-252088 | A | 01 October 1996 | (Family: none) | | | |
| WO | 2021/020554 | A1 | 04 February 2021 | US claims | 2022/0280601 | A1 | |
| | | | | EP | 4008349 | A1 | |
| | | | | CN | 114245749 | A | |
| WO | 2021/216618 | A1 | 28 October 2021 | EP claims | 4138869 | A1 | |
| | | | | KR | 10-2023-0004604 | A | |
| JP | 2009-269836 | A | 19 November 2009 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021216618 A
- JP 8252088 A